# EUROPEAN PATENT APPLICATION

(11) **EP 2 927 206 A1**
(43) Date of publication of application: **07.10.2015**
(21) Application number: 13858505.4
(22) Date of filing: 02.10.2013
(51) Int. Cl.: C07C 45/65, C07C 49/467, C07C 49/517, C07C 49/807, C07D 319/08, C07B 61/00

(54) **PRODUCTION METHOD FOR CARBONYL COMPOUND**

(30) Priority: 27.11.2012 JP 2012259165
(71) Applicant: Kureha Corporation, Chuo-ku Tokyo 103-8552 (JP)
(72) Inventor: KANNO, Hisashi, Tokyo 103-8552 (JP)
(74) Representative: Zimmermann & Partner Patentanwälte mbB
(86) International application number: PCT/JP2013/076865
(87) International publication number: WO 2014/083935

(57) **Abstract**

The present invention is to provide a method of producing a carbonyl compound at a higher yield. The method of producing a carbonyl compound according to the present invention produces a carbonyl compound represented by general formula (I) by subjecting a compound represented by general formula (II) to dealkoxycarbonylation in the presence of an organic carboxylic acid salt of a tertiary amine. 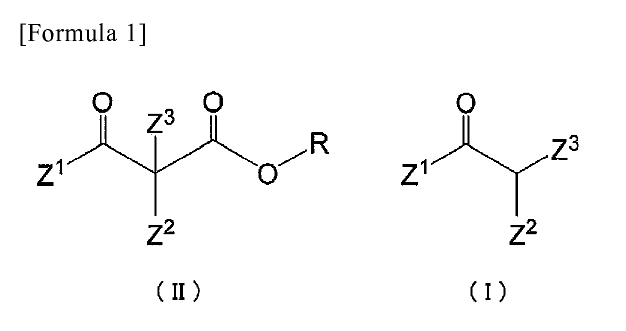 In the formula, R represents an alkyl group having from 1 to 4 carbons.

## Description

### TECHNICAL FIELD

The present invention relates to a method of producing a carbonyl compound, and particularly relates to a method of producing a carbonyl compound by subjecting a β-ketoester compound to dealkoxycarbonylation.

### BACKGROUND ART

Patent Document 1 describes a certain type of 2-(halogenated hydrocarbon substituted)-5-benzyl-1-azolylmethylcyclopentanol derivative as a compound that can be used as an active ingredient for agricultural and horticultural chemicals, industrial material protectants, and the like. Patent Document 1 also describes a method of producing a 2-benzyl-5,5-bis(hydroxymethyl)-cyclopentanone derivative having a protected hydroxy group from a 1-benzyl-2-oxocyclopentane carboxylic acid alkyl ester derivative, which is a β-ketoester compound, as a part of a step in the production method of this derivative.

### CITATION LIST

### Patent Literature

[Patent Document 1] WO/2011/070771 (published on June 16, 2011)

### SUMMARY OF INVENTION

### Technical Problem

In order to produce a 2-(halogenated hydrocarbon substituted)-5-benzyl-1-azolylmethylcyclopentanol derivative, which is used as an active ingredient of agricultural or horticultural chemicals and the like, at lower cost and in a large quantity, the yield of a 2-benzyl-5,5-bis(hydroxymethyl)-cyclopentanone derivative having a protected hydroxy group needs to be enhanced.

The present invention is completed in light of the problems described above. An object of the present invention is to provide a novel production method that can produce a carbonyl compound from β-ketoester compound at a higher yield.

### Solution to Problem

To solve the above problems, the method of producing a carbonyl compound according to the present invention is a method of producing a carbonyl compound represented by general formula (I) below from a compound represented by general formula (II) below, the method comprising a step of subjecting the compound represented by general formula (II) below to dealkoxycarbonylation in the presence of an organic carboxylic acid salt of a tertiary amine.

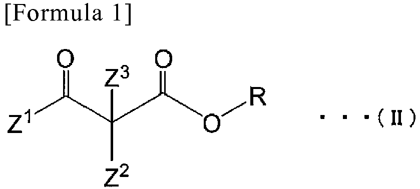

In general formula (II), Z¹ represents a substituted or unsubstituted alkyl group, cycloalkyl group, aryl group, or heterocyclic group; Z³ and Z² each independently represent a hydrogen atom, a substituted or unsubstituted alkyl group, cycloalkyl group, aryl group, or heterocyclic group; and R represents an alkyl group having from 1 to 4 carbons. Z¹ and Z² may be bonded to each other.

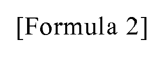

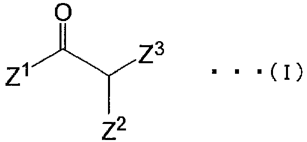

In general formula (I), Z¹, Z², and Z³ are the same as Z¹, Z², and Z³ in general formula (II) above, respectively.

### Advantageous Effects of Invention

By the method of producing a carbonyl compound according to the present invention, a carbonyl compound can be produced at a high yield from a β-ketoester compound.

### Description of Embodiments

As a result of diligent research, the present inventors have found that the yield of a 5-benzyl-2,2-bis(hydroxymethyl) cyclopentanone derivative having a protected hydroxy group can be enhanced by performing a reaction using an organic carboxylic acid salt of a tertiary amine in a production step for obtaining a 5-benzyl-2,2-bis(hydroxymethyl) cyclopentanone derivative having a protected hydroxy group from a 1-benzyl-3,3-bis(hydroxymethyl)-2-oxocyclopentane carboxylic acid alkyl ester derivative having a protected hydroxy group. In addition, the present inventors have found that a carbonyl compound can also be produced at a high yield by using an organic carboxylic acid salt of a tertiary amine in a reaction using another β-ketoester compound. As a result, the present invention has been completed.

An embodiment of the method of producing a carbonyl compound according to the present invention will be described hereinafter.

The method of producing a carbonyl compound according to the present invention is a method of producing a carbonyl compound represented by general formula (I) below from a β-ketoester compound (hereinafter, referred to as a "compound (II)") represented by general formula (II) below, wherein the method produces a carbonyl compound represented by general formula (I) by subjecting the compound (II) to dealkoxycarbonylation in the presence of an organic carboxylic acid salt of a tertiary amine.

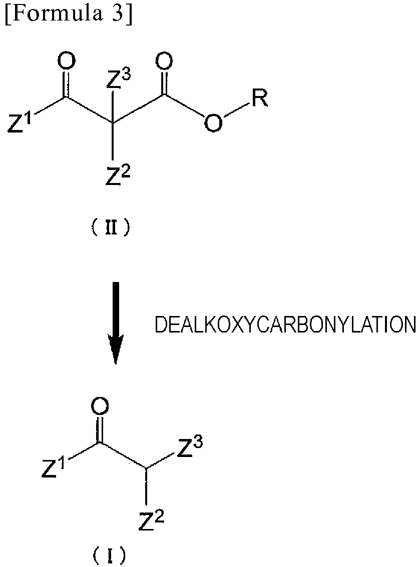

In the formula, Z¹ represents an alkyl group, cycloalkyl group, aryl group, or heterocyclic group, and the alkyl group, cycloalkyl group, aryl group, and heterocyclic group may have a substituent.

The number of carbons in the alkyl group of Z¹ is not particularly limited; however, the alkyl group can be exemplified by an alkyl group having from 1 to 8 carbons. Examples of the alkyl group having from 1 to 8 carbons include an ethyl group, methyl group, (1-methyl)ethyl group, n-propyl group, 1-methylpropyl group, 2-methylpropyl group, 1,1-dimethylethyl group, n-butyl group, n-pentyl group, n-hexyl group, n-heptyl group, n-octyl group, and the like.

The number of carbons constituting the ring of the cycloalkyl group of Z¹ is not particularly limited; however, the cycloalkyl group can be exemplified by a cycloalkyl group having from 3 to 6 carbons. Examples of the cycloalkyl group having from 3 to 6 carbons include a cyclopropyl group, cyclobutyl group, cyclopentyl group, cyclohexyl group, and the like.

Examples of the aryl group of Z¹ include a phenyl group, naphthyl group, indene group, azulene group, diphenyl group, and the like.

The number of atoms constituting the ring of a heterocyclic group of Z¹ is not particularly limited; however, the heterocyclic group can be exemplified by an aliphatic heterocyclic group having from 3 to 6 members and an aromatic heterocyclic group having from 5 or 6 members. Examples of a heterocyclic ring constituting the aliphatic heterocyclic ring having from 3 to 6 members include azetidine, aziridine, piperidine, piperazine, morpholine, pyrrolidine, oxetane, tetrahydrofuran, tetrahydropyran, and the like. Furthermore, examples of a heterocyclic ring constituting the aromatic heterocyclic group having from 5 to 6 members include thiophene, pyridine, thiazole, furan, pyrrole, oxazole, isoxazole, isothiazole, triazole, furazan, imidazole, pyrazole, pyrazine, pyrimidine, triazine, pyridazine, and the like. Examples thereof also include condensed heterocyclic rings, such as indole, benzofuran, benzothiophene, quinoline, quinoxaline; and the like.

Examples of the substituent that can be contained in the alkyl group, cycloalkyl group, aryl group, and heterocyclic group of Z¹ may have a halogen atom, an aliphatic hydrocarbon group, an aromatic hydrocarbon group, an aromatic heterocyclic group, an aliphatic hydrocarbon group in which at least one hydrogen atom is substituted with an aromatic hydrocarbon group or aromatic heterocyclic group, an alkoxy group, a carbonyloxyalkyl group, an amide group, a cyano group, a nitro group, and the like. Furthermore, a hydrogen atom in these substituents may be substituted with a halogen atom, hydroxy group, alkoxy group, alkyl group, haloalkyl group, aromatic hydrocarbon group, aromatic heterocyclic group, and the like. Furthermore, the hydroxy group may be protected by a protecting group that protects the hydroxy group.

Z² and Z³ each independently represent a hydrogen atom, alkyl group, cycloalkyl group, aryl group, or heterocyclic group, and the alkyl group, cycloalkyl group, aryl group, and heterocyclic group may have a substituent. Examples of the alkyl group, cycloalkyl group, aryl group, heterocyclic group, and the substituent that can be contained in these alkyl group, cycloalkyl group, aryl group, and heterocyclic group of Z² and Z³ are the same as the alkyl group, cycloalkyl group, aryl group, heterocyclic group, and the substituent that can be contained in these alkyl group, cycloalkyl group, aryl group, and heterocyclic group of Z¹ respectively.

Z² and Z³ may be the same or different from each other.

Z¹ and Z² may be bonded to each other and, together with a carbon atom to which Z¹ is bonded and a carbon atom to which Z² is bonded, form a ring.

R represents an alkyl group having from 1 to 4 carbons. Examples thereof include a methyl group, ethyl group, (1-methyl)ethyl group, n-propyl group, 1-methylpropyl group, 2-methylpropyl group, n-butyl group, 1,1-dimethylethyl group, and the like.

Examples of the tertiary amine constituting the organic carboxylic acid salt of a tertiary amine include aliphatic amines such as trimethylamine, triethylamine, ethyldimethylamine, tri-n-propylamine, triisopropylamine, tri-n-butylamine, triisobutylamine, tri-sec-butylamine, tri-tert-butylamine, tri-n-pentylamine, tri-n-hexylamine, tri-n-octylamine, diethylisopropylamine, diisopropylethylamine, tricyclopropylamine, tricyclobutylamine, tetramethylethylene diamine, tricyclopentylamine, and tricyclohexylamine; nitrogen-containing heterocyclic aliphatic amines such as N-methylpyrrolidine, N-ethylpyrrolidine, N-methylpiperidine, N-ethylpiperidine, N-n-butylpiperidine, N-methylhexamethylene imine, N-ethylhexamethylene imine, N-methylmorpholine, N-ethylmorpholine, N-butylmorpholine, N,N'-dimethylpiperazine, N,N'-diethylpiperazine, 1,5-diazabicyclo[4.3.1]non-5-ene, 1,4-diazabicyclo[2.2.2]octane, and 1,8-diazabicyclo[5.4.0]undec-7-ene; and nitrogen-containing heterocyclic aromatic amines such as pyridine, picoline, and lutidine. Among these, triethylamine, trimethylamine, ethyldimethylamine, N-methylpyrrolidine, pyridine, collidine, or picoline is preferred, triethylamine, trimethylamine, pyridine, or picoline is more preferred, triethylamine or pyridine is even more preferred, and triethylamine is particularly preferred.

As the organic carboxylic acid constituting the organic carboxylic acid salt of a tertiary amine, organic monocarboxylic acids are preferred, saturated or unsaturated aliphatic monocarboxylic acids are more preferred, and saturated aliphatic monocarboxylic acids are even more preferred. The numbers of carbons of the saturated aliphatic carboxylic acid is preferably from 1 to 8, and more preferably from 1 to 4. Examples of the organic carboxylic acid include formic acid, acetic acid, propionic acid, butyric acid, isobutyric acid, valeric acid, and isovaleric acid. Among these, formic acid, acetic acid, and propionic acid are preferred, and acetic acid is particularly preferred.

From the above facts, preferable specific examples of the organic carboxylic acid salt of a tertiary amine include acetate of trimethylamine, formic acid salt of triethylamine, acetate of trimethylamine, propionic acid salt of triethylamine, acetate of pyridine, acetate of 2-picoline, acetate of 3-picoline, acetate of 4-picoline, and the like. Among these, acetate of triethylamine or acetate of pyridine is preferred.

The amount of the organic carboxylic acid salt of a tertiary amine is, in terms of moles, for example, from 0.3 to 10 times, preferably from 0.5 to 5 times, and more preferably from 0.8 to 3 times, relative to the amount of the compound (II).

The organic carboxylic acid salt of a tertiary amine may be produced by adding the tertiary amine and the organic carboxylic acid to a reaction system. Addition of the tertiary amine and the organic carboxylic acid is not limited to separate addition of the tertiary amine and the organic carboxylic acid, and may be carried out by adding a premixed mixture of the tertiary amine and the organic carboxylic acid to a reaction system. The mixing ratio of the tertiary amine to the organic carboxylic acid may be 1:1. However, when a reaction is carried out for a compound in which the yield is expected to drop in a reaction under acidic conditions, as in the compound (IIc) and the compound (IId) to be described later, and the like, it is preferable to use a larger amount of tertiary amine than organic carboxylic acid in order to prevent excessive organic carboxylic acid, which results in an acidic reaction system. In this case, the amount of tertiary amine is, in terms of moles, for example, from 1.01 to 5 times, and preferably from 1.05 to 2 times, relative to the amount of organic carboxylic acid.

Additionally, an alkali metal salt of an organic carboxylic acid may be further added to the reaction system. When reaction progress is slow, the reaction can be accelerated by adding an alkali metal salt of an organic carboxylic acid, and the yield can be improved thereby. The alkali metal salt of an organic carboxylic acid may be added to the reaction system in advance, or may be added to the reaction system during the reaction.

Examples of the organic carboxylic acid constituting the alkali metal salt of an organic carboxylic acid are the same as the organic carboxylic acids constituting the organic carboxylic acid salt of a tertiary amine described above. Furthermore, the organic carboxylic acid constituting the alkali metal salt of an organic carboxylic acid may be the same as or different from the organic carboxylic acid constituting the organic carboxylic acid salt of a tertiary amine used in the reaction. However, it is preferable to use an organic carboxylic acid that is the same as that used in the reaction. Additionally, examples of the alkali metal constituting the alkali metal salt of an organic carboxylic acid include sodium and potassium. For example, when acetate of triethylamine is used as the organic carboxylic acid salt of a tertiary amine, sodium acetate and potassium acetate are preferred as the alkali metal salt of an organic carboxylic acid.

The dealkoxycarbonylation reaction may also be performed in a solvent. The solvent is not particularly limited as long as the solvent does not participate in the reaction. For example, amides such as N,N-dimethylformamide, N,N-dimethylacetamide, and N-methyl-2-pyrrolidone can be advantageously used. Furthermore, the solvent may be a mixed solvent with another solvent that can be mixed with these solvents (for example, toluene, DMSO, and the like).

The reaction temperature is, for example, from 0 to 250°C, preferably from room temperature to 200°C, and more preferably from 50 to 180°C. Furthermore, the reaction time is, for example, from 0.05 hours to a few days, preferably from 0.1 hours to 5 days, and more preferably from 0.5 hours to 2 days.

Note that, even when the reaction is not performed under inert gas, the reaction proceeds without problems. However, depending on the type of amine that is used, when the reaction takes place for a long time at a high temperature and when the reaction proceeds in the air, problems, such as coloration of the reaction liquid caused by being affected by air oxidation, may occur. In such a case, it is more preferable to perform the reaction under an inert gas (for example, nitrogen, argon, and the like) atmosphere.

Other reaction conditions can be easily designed and set by a person skilled in the art by referring to conventionally known production methods.

In the present specification, "dealkoxycarbonylation" indicates that -CO₂R is eventually substituted with a hydrogen atom. From the perspective of obtaining a carbonyl compound by removing -CO₂R from a β-ketoester compound, dealkoxycarbonylation is same as hydrolysis and decarboxylation of the β-ketoester compound. However, since, in the production method using organic carboxylic acid salt of a tertiary amine of the present invention, the reaction proceeds in the absence of water, the "dealkoxycarbonylation" in the present invention is clearly distinguished from a reaction including hydrolysis that requires water. Note that, when water is present in the reaction system, a hydroxide ion may be generated from the water molecule under a condition containing bases, thereby causing undesired side reactions. Therefore, it is preferred that the system contains no water.

In the production method according to the present invention, since the reaction is performed using a salt of a tertiary amine, but not an acid or alkali, it is possible to perform the reaction under a neutral or almost neutral condition. Therefore, even for a compound which is unstable to an acid or alkali and the yield of which is anticipated to be lowered under acidic conditions or basic conditions, the reaction can be performed with a high yield.

The reaction mechanism of the dealkoxycarbonylation in the method of producing a carbonyl compound according to the present invention is presumed to include the following reaction mechanism, for example.
First, an acid anhydride is produced by replacing the alkoxy group in the compound (II) with an organic carboxylate anion. Because the carboxylic acid salt of a tertiary amine is a proton source, the alkoxy group produced by replacement becomes alkyl alcohol, and a β-ketocarboxylic acid is produced by a reaction between the alkyl alcohol and the acid anhydride. A compound represented by general formula (I) is formed by decarboxylation of the produced β-ketocarboxylic acid. Meanwhile, since the organic carboxylic acid alkyl ester produced by the substitution has a relatively low boiling point, it is removed to outside the system.

However, in the method of producing a carbonyl compound according to the present invention, the reaction mechanism is not limited to the above reaction mechanism or other specific reaction mechanisms as long as the reaction proceeds using an organic carboxylic acid salt of a tertiary amine. Furthermore, the method is not limited to cases where the reaction proceeds by a single reaction mechanism, and the method may be a method in which the reaction proceeds by a plurality of reaction mechanisms.

A preferable aspect of the method of producing a carbonyl compound according to the present invention is a method of producing a carbonyl compound represented by general formula (Ia). The method produces a carbonyl compound represented by general formula (Ia) by subjecting a β-ketoester compound having a cyclopentane ring represented by general formula (IIa) below (hereinafter, referred to as a "compound (IIa)") to dealkoxycarbonylation in the presence of an organic carboxylic acid salt of a tertiary amine.

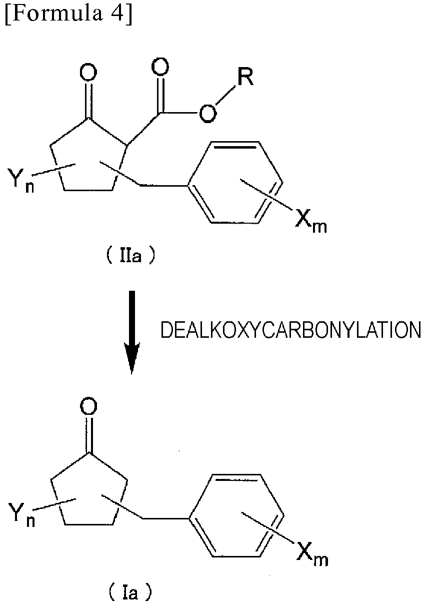

In the formula, Y represents an alkyl group or haloalkyl group having from 1 to 6 carbons, an alkenyl group or haloalkenyl group having from 2 to 6 carbons, an alkynyl group or haloalkynyl group having from 2 to 6 carbons, or a group in which a part of hydrogen atoms of the alkyl group, haloalkyl group, alkenyl group, haloalkenyl group, alkynyl group, or haloalkynyl group is substituted with -OG (G represents a protecting group of a hydroxy group).

Examples of the alkyl group having from 1 to 6 carbons include a methyl group, ethyl group, (1-methyl)ethyl group, n-propyl group, 1-methylpropyl group, 2-methylpropyl group, n-butyl group, 1-methylbutyl group, 2-methylbutyl group, 1-ethylpropyl group, 1,1-dimethylethyl group, and the like. Among these, an alkyl group having from 1 to 4 carbons is preferable. A methyl group and an ethyl group are more preferable, and a methyl group is even more preferable.

Examples of the haloalkyl group having from 1 to 6 carbons include a chloromethyl group, dichloromethyl group, trichloromethyl group, 2-chloroethyl group, 1-chloroethyl group, 2,2-dichloroethyl group, 1,2-dichloroethyl group, 2,2,2-trichloroethyl group, 3-chloropropyl group, 2,3-dichloropropyl group, 1-chloro-1-methylethyl group, 2-chloro-1-methylethyl group, 2-chloropropyl group, 4-chlorobutyl group, 5-chloropentyl group, fluoromethyl group, difluoromethyl group, trifluoromethyl group, 2-fluoroethyl group, 1-fluoroethyl group, 2,2-difluoroethyl group, 1,2-difluoroethyl group, 2,2,2-trifluoroethyl group, 3-fluoropropyl group, 2,3-difluoropropyl group, 1-fluoro-1-methylethyl group, 2-fluoro-1-methylethyl group, 2-fluoropropyl group, 3,3,3-trifluoropropyl group, 2,2,3,3-tetrafluoropropyl group, 2,2,3,3,3-pentafluoropropyl group, 4-fluorobutyl group, 5-fluoropentyl group, bromomethyl group, dibromomethyl group, tribromomethyl group, 2-bromoethyl group, 1-bromoethyl group, 2,2-dibromoethyl group, 1,2-dibromoethyl group, 2,2,2-tribromoethyl group, 3-bromopropyl group, 2,3-dibromopropyl group, 1-bromo-1-methylethyl group, 2-bromo-1-methylethyl group, 2-bromopropyl group, 4-bromobutyl group, 5-bromopentyl group, iodomethyl group, diiodomethyl group, 2-iodoethyl group, 1-iodoethyl group, 2,2-diiodoethyl group, 1,2-diiodoethyl group, 2,2,2-triiodoethyl group, 3-iodopropyl group, 2,3-diiodopropyl group, 1-iodo-1-methylethyl group, 2-iodo-1-methylethyl group, 2-iodopropyl group, 4-iodobutyl group, and the like. Among these, a haloalkyl group having from 1 to 4 carbons is preferable, and a haloalkyl group having from 1 to 3 carbons is more preferable.

Examples of the alkenyl group having from 2 to 6 carbons include an ethenyl group, 1,2-dimethylethenyl group, 4-methyl-1,3-butadienyl group, 1-propenyl group, 2-propenyl group, 2-methyl-2-propenyl group, 3-methyl-2-propenyl group, 2-butenyl group, 3-butenyl group, 3-methyl-3-butenyl group, and the like. Among these, an alkenyl group having from 2 to 4 carbons is preferable.

Examples of the haloalkenyl group having from 2 to 6 carbons include a 2-chloroethenyl group, 2,2-dichloroethenyl group, 2-chloro-2-propenyl group, 3,3-dichloro-2-propenyl group, 2,3-dichloro-2-propenyl group, 3,3-dichloro-2-methyl-2-propenyl group, 3-chloro-2-butenyl group, 2-fluoroethenyl group, 2,2-difluoroethenyl group, 2-fluoro-2-propenyl group, 3,3-difluoro-2-propenyl group, 2,3-difluoro-2-propenyl group, 3,3-difluoro-2-methyl-2-propenyl group, 3-fluoro-2-butenyl group, 2-bromoethenyl group, 2,2-dibromoethenyl group, 2-bromo-2-propenyl group, 3,3-dibromo-2-propenyl group, 2,3-dibromo-2-propenyl group, 3,3-dibromo-2-methyl-2-propenyl group, 3-bromo-2-butenyl group, 2-iodoethenyl group, 2,2-diiodoethenyl group, 2-iodo-2-propenyl group, 3,3-diiodo-2-propenyl group, 2,3-diiodo-2-propenyl group, and the like. Among these, a haloalkenyl group having from 2 to 4 carbons is preferable.

Examples of the alkynyl group having from 2 to 6 carbons include an ethynyl group, 1-propynyl group, 2-propynyl group, 1-butynyl group, 2-butynyl group, and the like. Among these, an alkynyl group having from 2 to 4 carbons are preferable.

Examples of the haloalkynyl group having from 2 to 6 carbons include a 2-fluoroethynyl group, 2-chloroethynyl group, 3-fluoro-2-propynyl group, 3-chloro-2-propynyl group, 3-bromo-2-propynyl group, and the like. Among these, a haloalkynyl group having from 2 to 4 carbons is preferable.

The protecting group G in -OG is a protecting group that protects a hydroxy group. The protecting group is not particularly limited as long as the protecting group dissociates under a proper condition to form a hydroxy group. Examples of the protecting group G include a protecting group that dissociates under acidic conditions, a protecting group that splits under reducing conditions such as a hydrogenation reaction, and the like.

n represents an integer from 0 to 6, and n is preferably from 0 to 3, and more preferably from 0 to 2. When n is 2 or greater, a plurality of Y may be the same or different each other. Furthermore, when n is 2 or greater, two Y may be bonded to one carbon atom. Furthermore, when n is 2 or greater, a plurality of Y may be bonded to each other and, together with carbon atom(s) to which the plurality of Y are bonded, form a ring;

X represents a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group.

Examples of the halogen atom of X include a fluorine atom, chlorine atom, bromine atom, iodine atom, and the like. Among these, a fluorine atom, chlorine atom, and bromine atom are preferable, and a fluorine atom and chlorine atom are more preferable.

Specific examples of the alkyl group having from 1 to 4 carbons of X include a methyl group, ethyl group, n-propyl group, isopropyl group, n-butyl group, isobutyl group, sec-butyl group, and tert-butyl group. Among these, an alkyl group having from 1 to 3 carbons is preferable. An alkyl group having from 1 to 2 carbons are more preferable, and a methyl group is even more preferable.

Examples of the haloalkyl group having from 1 to 4 carbons of X include a dichloromethyl group, trichloromethyl group, 2-chloroethyl group, 1-chloroethyl group, 2,2-dichloroethyl group, 1,2-dichloroethyl group, 2,2,2-trichloroethyl group, 3-chloropropyl group, 2,3-dichloropropyl group, 1-chloro-1-methylethyl group, 2-chloro-1-methylethyl group, 2-chloropropyl group, 4-chlorobutyl group, fluoromethyl group, difluoromethyl group, trifluoromethyl group, 2-fluoroethyl group, 1-fluoroethyl group, 2,2-difluoroethyl group, 1,2-difluoroethyl group, 2,2,2-trifluoroethyl group, 3-fluoropropyl group, 2,3-difluoropropyl group, 1-fluoro-1-methylethyl group, 2-fluoro-1-methylethyl group, 2-fluoropropyl group, 3,3,3-trifluoropropyl group, 2,2,3,3-tetrafluoropropyl group, 2,2,3,3,3-pentafluoropropyl group, 4-fluorobutyl group, dibromomethyl group, tribromomethyl group, 2-bromoethyl group, 2,2-dibromoethyl group, 1,2-dibromoethyl group, 2,2,2-tribromoethyl group, 3-bromopropyl group, 2,3-dibromopropyl group, 1-bromo-1-methylethyl group, 2-bromo-1-methylethyl group, 2-bromopropyl group, diiodomethyl group, 2,2-diiodoethyl group, 1,2-diiodoethyl group, 2,2,2-triiodoethyl group, 2,3-diiodopropyl group, 1-iodo-1-methylethyl group, 2-iodo-1-methylethyl group, and the like. Among these, a haloalkyl group having from 1 to 3 carbons are preferable, and a haloalkyl group having 1 or 2 carbons is more preferable. A trihaloalkyl group having 1 carbon is even more preferable.

Examples of the alkoxy group having from 1 to 4 carbons in X include a methoxy group, ethoxy group, n-propoxy group, and the like. Among these, an alkoxy group having from 1 to 3 carbons is preferable, and an alkoxy group having 1 or 2 carbons is more preferable. A methoxy group is even more preferable.

Examples of the haloalkoxy group having from 1 to 4 carbons of X include a trifluoromethoxy group, difluoromethoxy group, 1,1,2,2,2-pentafluoroethoxy group, 2,2-tifluoroethoxy group, 2-tifluoroethoxy group, and the like. Among these, a haloalkoxy group having from 1 to 3 carbons is preferable, and a haloalkoxy group having 1 or 2 carbons is more preferable. A dihalomethoxy group and trihalomethoxy group having 1 carbon are even more preferable.

m represents an integer from 0 to 5. m is preferably an integer from 0 to 3, more preferably an integer from 0 to 2, and even more preferably 0 or 1. When m is 2 or greater, a plurality of X may be the same or different each other. When m is 1 or more, X may be positioned at any one of 2 to 6 positions of the benzene ring; however, when m is 1, X is preferably at a position that forms a 4-substituted benzyl.

R is the same as R in general formula (II) above.

The type and used amount of the organic carboxylic acid salt of a tertiary amine, the type of solvent, the reaction conditions of the reaction, and the like used in the reaction that dealkoxycarbonylates the compound (IIa) are the same as the above-described type and used amount of the organic carboxylic acid salt of a tertiary amine, type of solvent, reaction conditions, and the like.

Preferable examples of the compound (IIa) include a compound represented by general formula (IIa-1) below and a compound represented by general formula (IIa-2) below.

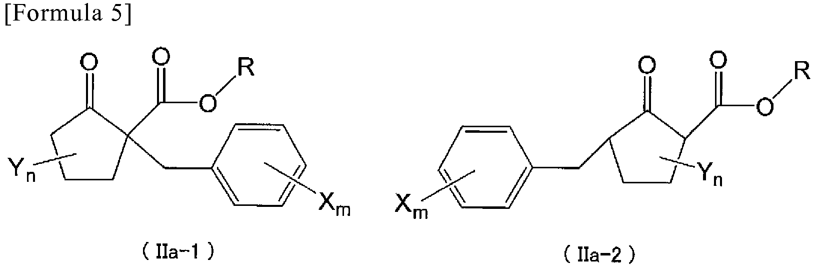

A more preferable aspect of the method of producing a carbonyl compound according to the present invention is a method of producing a carbonyl compound represented by general formula (Ib). The method produces a carbonyl compound represented by general formula (Ib) by subjecting a β-ketoester compound having a cyclopentane ring represented by general formula (IIb) below (hereinafter, referred to as "compound (IIb)") to dealkoxycarbonylation in the presence of an organic carboxylic acid salt of a tertiary amine.

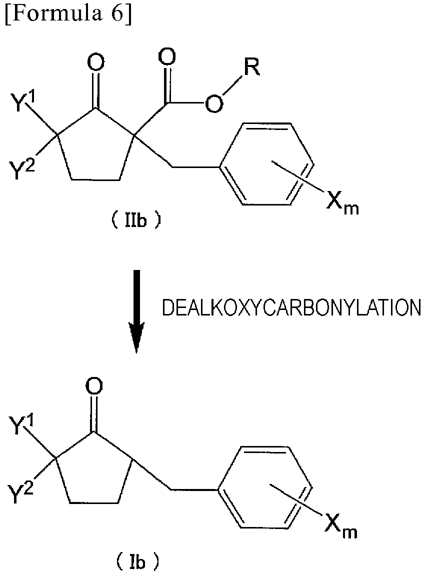

In the formula, Y¹ and Y² each independently represent an alkyl group or haloalkyl group having from 1 to 6 carbons, or a group in which a part of hydrogen atoms of the alkyl group or haloalkyl group are substituted with -OG¹ (G¹ represents a protecting group of a hydroxy group).

Examples of the alkyl group and haloalkyl group having from 1 to 6 carbons of Y¹ and Y² are the same as those for the alkyl group and haloalkyl group having from 1 to 6 carbons of Y described above.

The protecting group G¹ is a protecting group that protects a hydroxy group. The protecting group is not particularly limited as long as the protecting group dissociates under a proper condition to form a hydroxy group. Examples of the protecting group G¹ include a protecting group that dissociates under acidic conditions. Examples of the protecting group that dissociates under acidic conditions include an alkoxymethyl group and alkoxyethyl group in which alkoxy moiety has from 1 to 4 carbons (for examaple, methoxymethyl group, ethoxymethyl group, and the like), an alkyl group having from 1 to 4 carbons (for example, methyl group, ethyl group, t-butyl group, and the like), a substituted or unsubstituted benzyl group, a substituted or unsubstituted tetrahydropyranyl group, a substituted or unsubstituted tetrahydrofuranyl group, an allyl group, a silyl group (for example, triethylsilyl group, t-butyldimethylsilyl group, and the like), and the like. Furthermore, the protecting group for two hydroxy groups may be bonded to each other; for example, and a case where two hydroxy groups are protected at the same time by acetals, such as methylene acetal or ethylidene acetal, is possible.

Furthermore, examples thereof also include a protecting group that splits under reducing conditions. Examples of the protecting group that splits under reducing conditions include a benzyl group, a substituted or unsubstituted benzyl group, such as a p-methoxybenzyl group, and the like.

Y¹ and Y² may be bonded to each other and, together with a carbon atom to which Y¹ and Y² are bonded, form a ring.

Furthermore, R, X, and m are the same as R, X, and m in general formula (II) above.

The type and used amount of the organic carboxylic acid salt of a tertiary amine, the type of solvent, the reaction conditions of the reaction, and the like used in the reaction that dealkoxycarbonylates the compound (IIb) are the same as the above-described type and used amount of the organic carboxylic acid salt of a tertiary amine, type of solvent, reaction conditions, and the like.

Another preferable aspect of the method of producing a carbonyl compound according to the present invention is a method of producing a carbonyl compound represented by general formula (Ic) (hereinafter, referred to as "compound (Ic)"). The method produces a compound (Ic) by subjecting a β-ketoester compound having a cyclopentane ring represented by general formula (IIc) below (hereinafter, referred to as "compound (IIc)") to dealkoxycarbonylation in the presence of an organic carboxylic acid salt of a tertiary amine.

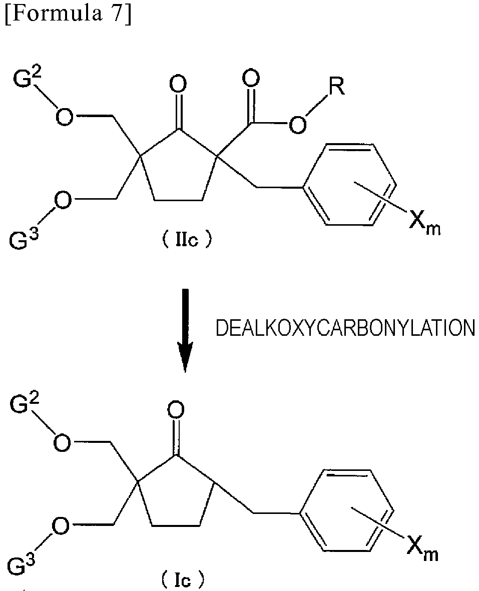

In the formula, G² and G³ each independently represent a protective group that protects a hydroxy group, and, in particular, represents a protective group that dissociates under acidic conditions. Note that G² and G³ may be bonded to each other and, together with oxygen atoms to which, respectively, G² and G³ are bonded, carbon atoms to which, respectively, the oxygen atoms are bonded, and a carbon atom of the cyclopentane ring to which these carbon atoms are bonded, form a ring.

Examples of the protecting group in cases where G² and G³ are not bonded to each other include an alkoxymethyl group in which an alkoxy moiety has from 1 to 4 carbons (for example, methoxymethyl group, ethoxymethyl group, and the like), an alkoxyethyl group in which an alkoxy moiety has from 1 to 4 carbons (for example, 1-ethoxyethyl group, 1-methyl-1-methoxyethyl group, and the like), an alkyl group having from 1 to 4 carbons (for example, t-butyl group, methyl group, and the like), a substituted or unsubstituted benzyl group, a substituted or unsubstituted tetrahydropyranyl group, a substituted or unsubstituted tetrahydrofuranyl group, an allyl group, and a silyl group (for example, triethylsilyl group, t-butyldimethylsilyl group, and the like).

On the other hand, examples of the protecting group in cases where G² and G³ are bonded to each other include methylene acetal, ethylidene acetal, t-butylmethylidene ketal, 1-t-butylethylidene ketal, 1-phenylethylidene ketal, acrolein acetal, isopropylidene ketal (acetonide), cyclopentylidene ketal, cyclohexylidene ketal, cycloheptylidene ketal, benzylidene acetal, p-methoxybenzylidene acetal, 2,4-dimethoxybenzylidene ketal, 3,4-dimethoxybenzylidene ketal, 2-nitrobenzylidene acetal, 4-nitrobenzylidene acetal, mesitylene acetal, 1-naphthaldehyde acetal, benzophenone ketal, camphor ketal, menthone ketal, methoxymethylene acetal, ethoxymethylene acetal, dimethoxymethylene orthoester, 1-methoxyethylidene orthoester, 1-ethoxyethylidene orthoester, methylidene orthoester, phthalide orthoester, 1,2-dimethoxyethylidene orthoester, α-methoxybenzylidene orthoester, 2-oxacyclopentylidene orthoester, butane-2,3-bis-acetal, cyclohexane-1,2-diacetal, bis-dihydropyran ketal, di-t-butylsilylene, 1,3-(1,1,3,3-tetraisopropyl)disiloxanilidene, and 1,1,3,3-tetra-t-butoxydisiloxanilidene.

Furthermore, R, X, and m are the same as R, X, and m in general formula (II) above.

The type and used amount of the organic carboxylic acid salt of a tertiary amine, the type of solvent, the reaction conditions of the reaction, and the like used in the reaction that dealkoxycarbonylates the compound (IIc) are the same as the above-described type and used amount of the organic carboxylic acid salt of a tertiary amine, type of solvent, reaction conditions, and the like.

G² and G³ are protecting groups that dissociate under acidic conditions. Therefore, when hydrolysis and decarboxylation reactions are used in an attempt to obtain the compound (Ic) from the compound (IIc), the yield of the compound (Ic) decreases if the reaction is performed under acidic conditions. Meanwhile, when the inventors of the present application conducted various investigations, it was found that, when the compound (IIc) was hydrolyzed/decarboxylated under basic conditions, the yield of the compound (Ic) decreased due to the occurrence of side reactions involving the opening of cyclopentane rings. Therefore, for a reaction to obtain the compound (Ic) from the compound (IIc), performing the reaction under a neutral or almost neutral condition is desired. Here, according to the production method of the present invention in which dealkoxycarbonylation is performed using an organic carboxylic acid salt of a tertiary amine, it is possible to perform the reaction under a neutral or almost neutral condition. Therefore, the production method of the present invention in which dealkoxycarbonylation is performed using an organic carboxylic acid salt of a tertiary amine exhibits excellent effect especially in a method of producing the compound (Ic) from the compound (IIc) for which performing the reaction under a neutral or almost neutral condition is desired.

Furthermore, a side reaction in which a cyclopentane ring is opened upon performing hydrolysis/decarboxylation under basic conditions can occur not only in a reaction for the compound (IIc) but also in reactions for the compound (IIa) and the compound (IIb) in which a cyclopentane ring is contained. Therefore, when it is not desirable to perform hydrolysis/decarboxylation of the compound (IIa) and the compound (IIb) under acidic conditions due to some reason (for example, in cases where the yield will be decreased by performing hydrolysis/decarboxylation under acidic conditions), the production method of the present invention in which dealkoxycarbonylation is performed using an organic carboxylic acid salt of a tertiary amine is particularly preferably employed.

A preferable aspect in cases where G² and G³ are bonded to each other to form a ring is a method of producing a carbonyl compound represented by general formula (Id). The method produces a carbonyl compound represented by general formula (Id) by subjecting a β-ketoester compound having a cyclopentane ring represented by general formula (IId) below to dealkoxycarbonylation in the presence of an organic carboxylic acid salt of a tertiary amine.

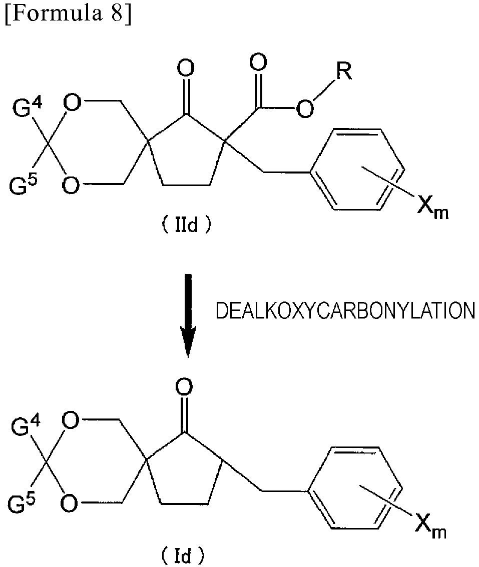

In the formula, G⁴ and G⁵ each independently represent a hydrogen atom, an alkyl group having from 1 to 4 carbons, an alkenyl group having from 1 to 4 carbons, a phenyl group, a naphthyl group, or a benzyl group. A phenyl moiety of the phenyl group, naphthyl group, and benzyl group of G⁴ and G⁵ may be substituted with an alkyl group having from 1 to 4 carbons (for example, methyl group, ethyl group, and the like); an alkoxy group having from 1 to 4 carbons (for example, methoxy group, ethoxy group, and the like); a nitro group; or a halogen atom (for example, fluorine atom, chlorine atom, and the like). Note that G⁴ and G⁵ may be bonded to each other and, together with a carbon atom to which G⁴ and G⁵ are bonded, form a ring. Among these, G⁴ and G⁵ are more preferably each independently a hydrogen atom or an alkyl group having from 1 to 4 carbons, such as a methyl group, ethyl group, and n-propyl group; and even more preferably each independently a hydrogen atom, methyl group, or ethyl group. Furthermore, both of G⁴ and G⁵ are particularly preferably a methyl group.

Furthermore, R, X, and m are the same as R, X, and m in general formula (II) above.

The type and used amount of the organic carboxylic acid salt of a tertiary amine, the type of solvent, the reaction conditions of the reaction, and the like used in the reaction that dealkoxycarbonylates the compound (IId) are the same as the above-described type and used amount of the organic carboxylic acid salt of a tertiary amine, type of solvent, reaction conditions, and the like.

A particularly preferable aspect of the method of producing a carbonyl compound according to the present invention is a method of producing a carbonyl compound represented by general formula (Ie). The method produces a carbonyl compound represented by general formula (Ie) by subjecting a β-ketoester compound having a cyclopentane ring represented by general formula (IIe) below to dealkoxycarbonylation in the presence of an organic carboxylic acid salt of a tertiary amine.

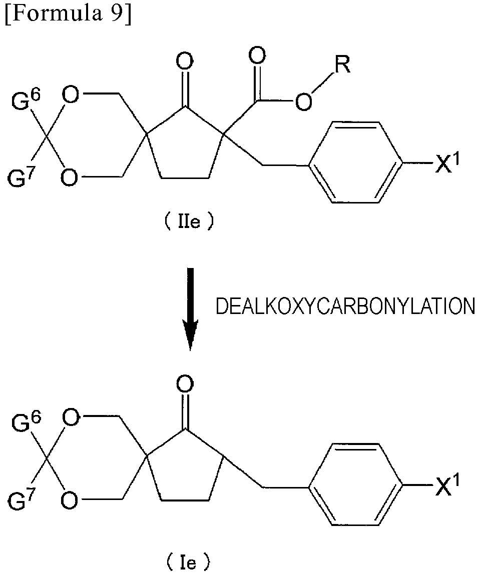

In the formula, G⁶ and G³ each independently represent a hydrogen atom or an alkyl group having from 1 to 4 carbons, such as a methyl group, ethyl group, and n-propyl group. Among these, G⁶ and G⁷ are preferably each independently a hydrogen atom, methyl group, or ethyl group. Furthermore, both of G⁶ and G³ are more preferably a methyl group.

X¹ represents a hydrogen atom, chlorine atom, or fluorine atom.

### (Summary)

The method of producing a carbonyl compound according to the present invention is a method of producing a carbonyl compound represented by general formula (I) below from a compound represented by general formula (II) below, the method comprising a step of subjecting the compound represented by general formula (II) below to dealkoxycarbonylation in the presence of an organic carboxylic acid salt of a tertiary amine.

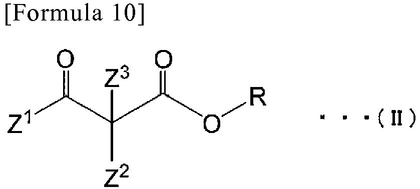

In general formula (II), Z¹ represents a substituted or unsubstituted alkyl group, cycloalkyl group, aryl group, or heterocyclic group; Z³ and Z² each independently represent a hydrogen atom, a substituted or unsubstituted alkyl group, cycloalkyl group, aryl group, or heterocyclic group; and R represents an alkyl group having from 1 to 4 carbons. Z¹ and Z² may be bonded to each other.

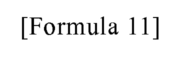

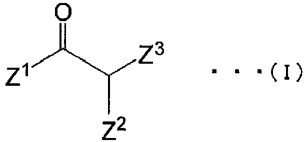

In general formula (I), Z¹, Z², and Z³ are the same as Z¹, Z², and Z³ in general formula (II) above, respectively.

Furthermore, in the method of producing a carbonyl compound according to the present invention, a tertiary amine constituting the organic carboxylic acid salt of a tertiary amine is preferably triethylamine, trimethylamine, pyridine, or picoline.

Furthermore, in the method of producing a carbonyl compound according to the present invention, an organic carboxylic acid constituting the organic carboxylic acid salt of a tertiary amine is preferably an aliphatic monocarboxylic acid.

Furthermore, in the method of producing a carbonyl compound according to the present invention, an organic carboxylic acid salt of a tertiary amine is preferably acetate of triethylamine or pyridine.

Furthermore, in the method of producing a carbonyl compound according to the present invention, an alkali metal salt of an organic carboxylic acid is preferably further added to the reaction system.

Furthermore, in the method of producing a carbonyl compound according to the present invention, the compound represented by general formula (II) above is preferably a compound represented by general formula (IIa) below; and the carbonyl compound represented by general formula (I) above is preferably a carbonyl compound represented by general formula (Ia) below.

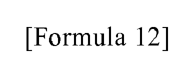

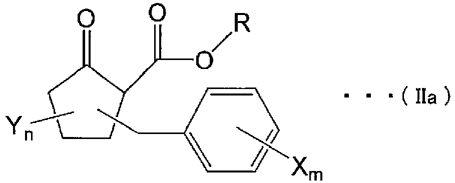

In general formula (IIa), R represents an alkyl group having from 1 to 4 carbons, Y represents an alkyl group or haloalkyl group having from 1 to 6 carbons, an alkenyl group or haloalkenyl group having from 2 to 6 carbons, an alkynyl group or haloalkynyl group having from 2 to 6 carbons, or a group in which a part of hydrogen atoms of the alkyl group, haloalkyl group, alkenyl group, haloalkenyl group, alkynyl group, or haloalkynyl group is substituted with -OG (G represents a protecting group of a hydroxy group); and n is an integer from 0 to 6. When n is 2 or greater, a plurality of Y may be the same or different. When n is 2 or greater, a plurality of Y may be bonded to each other and, together with carbon atom(s) to which the plurality of Y are bonded, form a ring. X represents a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; and m is an integer from 0 to 5, when m is 2 or greater, a plurality of X may be the same or different.

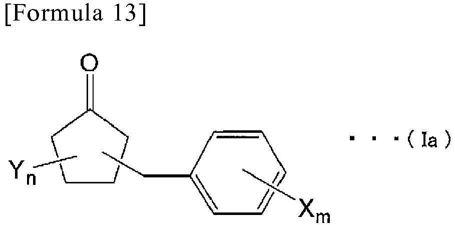

In general formula (Ia), X, Y, m, and n are the same as X, Y, m, and n in general formula (IIa) above, respectively.

Furthermore, in the method of producing a carbonyl compound according to the present invention, the compound represented by general formula (IIa) above is preferably a compound represented by general formula (IIb) below; and the carbonyl compound represented by general formula (Ia) above is preferably a carbonyl compound represented by general formula (Ib) below:

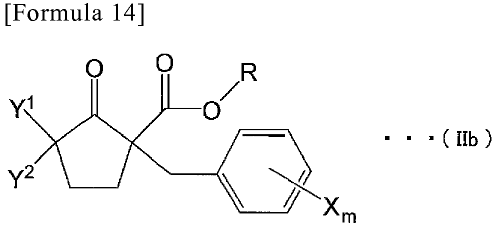

In general formula (IIb), Y¹ and Y² each independently represent an alkyl group or haloalkyl group having from 1 to 6 carbons, or a group in which a part of hydrogen atoms of the alkyl group or haloalkyl group is substituted with -OG¹ (G¹ represents a protecting group of a hydroxy group), and Y¹ and Y² may be bonded to each other and, together with a carbon atom to which Y¹ and Y² are bonded, form a ring. R, X, and m are the same as R, X, and m in general formula (IIa) above, respectively.

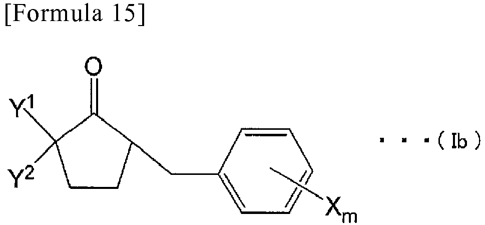

In general formula (Ib), X, Y¹, Y², and m are the same as X, Y¹, Y², and m in general formula (IIb) above, respectively.

Furthermore, in the method of producing a carbonyl compound according to the present invention, the compound represented by general formula (IIb) above is preferably a compound represented by general formula (IIc) below; and the carbonyl compound represented by general formula (Ib) above is preferably a carbonyl compound represented by general formula (Ic) below.

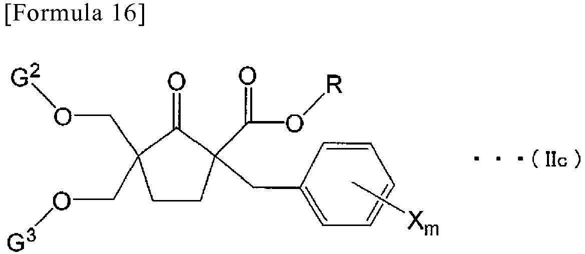

In general formula (IIc), G² and G³ each independently represent a protecting group that dissociates under acidic conditions, and G² and G³ may be bonded to each other. R, X, and m are the same as R, X, and m in general formula (IIa) above, respectively.

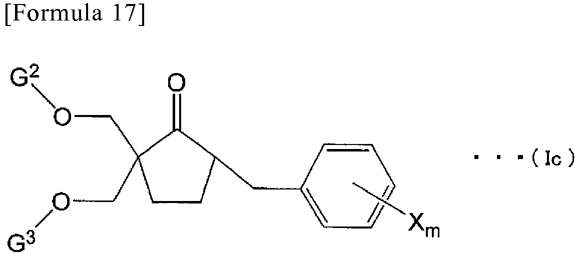

In general formula (Ic), X, G², G³, and m are the same as X, G², G³, and m in general formula (IIc) above, respectively.

Furthermore, in the method of producing a carbonyl compound according to the present invention, it is preferable if the organic carboxylic acid salt of a tertiary amine is produced in the reaction system by adding the tertiary amine to the compound represented by general formula (IIc) above and then adding an organic carboxylic acid in an amount smaller than the added amount of the tertiary amine, and dealkoxycarbonylation is performed.

Furthermore, in the method of producing a carbonyl compound according to the present invention, the compound represented by general formula (IIc) above is preferably a compound represented by general formula (IId) below; and the carbonyl compound represented by general formula (Ic) above is preferably a carbonyl compound represented by general formula (Id) below:

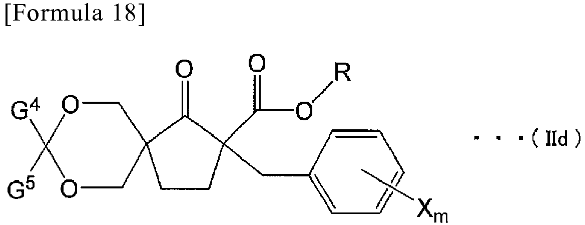

In general formula (IId), G⁴ and G⁵ each independently represent a hydrogen atom, an alkyl group having from 1 to 4 carbons, an alkenyl group having from 1 to 4 carbons, a substituted or unsubstituted phenyl group, naphthyl group, or benzyl group, and G⁴ and G⁵ may be bonded to each other and, together with a carbon atom to which G⁴ and G⁵ are bonded, form a ring. R, X, and m are the same as R, X, and m in general formula (IIa) above, respectively.

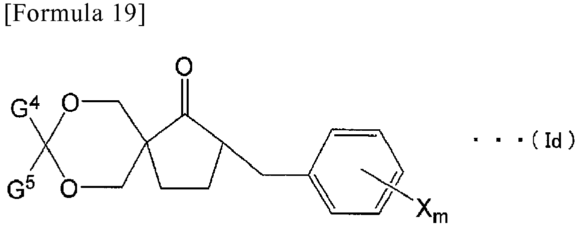

In general formula (Id), X, G⁴, G⁵, and m are the same as X, G⁴, G⁵, and m in general formula (IId) above, respectively.

Furthermore, in the method of producing a carbonyl compound according to the present invention, G⁴ and G⁵ are preferably each independently a hydrogen atom or an alkyl group having from 1 to 4 carbons.

Furthermore, in the method of producing a carbonyl compound according to the present invention, m is preferably an integer from 0 to 2; and when m is 1 or 2, X is preferably a halogen atom.

Embodiments of the present invention will be described in further detail hereinafter using practical examples. Of course, the present invention is not limited to the practical examples below, and it goes without saying that various modes are possible with regard to the details thereof. Furthermore, the present invention is not limited to the embodiments described above, and various modifications are possible within the scope indicated in the claims. Embodiments obtained by appropriately combining the technical means disclosed by the embodiments are also included in the technical scope of the present invention. In addition, all of the documents cited in this specification are hereby incorporated by reference.

### EXAMPLES

### Working Example 1: Synthesis of 5-(4-chlorobenzyl)-2,2-bis((methoxymethoxy)methyl) cyclopentanone

Dimethylacetamide (1 mL), triethylamine (1.5 mL, 0.0482 × 2.2 mol), and acetic acid (0.58 mL, 0.0482 × 2.1 mol) were added to 1-(4-chlorobenzyl)-3,3-bis((methoxymethoxy)methyl)-2-oxocyclopentane carboxylic acid methyl ester (2.0 g, 0.00482 mol), an isobaric dropping funnel was mounted between a cooling condenser and the reaction vessel, and they were stirred at 155°C for 5 hours such that the distillation product did not return, and then they were reacted at 165°C for 18 hours. In the reaction liquid, ethyl acetate and saturated sodium bicarbonate water were added, and the mixture was partitioned. After extracting the aqueous layer using ethyl acetate, the organic layer was dried using anhydrous sodium sulfate and concentrated. Thereafter, the dried and concentrated product was purified using a silica gel column, and a target product was obtained.

The yield in grams was 1.53 g, and the percent yield was 89%.

### Working Example 2: Synthesis of 2-(4-chlorobenzyl)-8,8-dimethyl-7,9-dioxaspiro[4.5]decan-1-one

Dimethylacetamide (1 mL) and triethylamine (1.7 mL, 0.00545 × 2.25 mol) were added to 2-(4-chlorobenzyl)-8,8-dimethyl-1-oxo-7,9-dioxaspiro[4.5]decane-2-carboxylic acid methyl ester (2.0 g, 0.00546 mol), and acetic acid (0.63 mL, 0.00545 × 2.0 mol) was also added, after which an isobaric dropping funnel was mounted between a cooling condenser and the reaction vessel, and they were stirred at 155°C for 11 hours such that the distillation product did not return. Toluene was added to the reaction solution, and after washing with saturated sodium bicarbonate water and saturated brine solution, the aqueous layer was extracted with toluene. The organic layer was dried with anhydrous sodium sulfate and concentrated, and then the resulting substance was purified by silica gel column chromatography to obtain the target product.

The yield in grams was 1.59 g, and the percent yield was 94%.

Working Example 3: Synthesis of 5-(4-chlorobenzyl)-2,2-dimethyl cyclopentanone Dimethylacetamide (1.5 mL), sodium acetate (0.042 g, 0.00509 × 0.1 mol), and triethylamine (1.2 mL, 0.00509 × 1.7 mol) were added to 2-(4-chlorobenzyl)-l-oxo-7,9-dioxaspiro[4.5]decane-2-carboxylic acid methyl ester (1.5 g, 0.00509 mol), and then acetic acid (0.44 mL, 0.00509 × 1.5 mol) was added. An isobaric dropping funnel was mounted between a cooling condenser and the reaction vessel, and they were stirred at 165°C for 24 hours such that the distillation product did not return. Toluene and saturated sodium bicarbonate water were added to the reaction solution, and after the mixture was partitioned, the aqueous layer was extracted with toluene. The organic layer was dried with anhydrous sodium sulfate and concentrated, and then the resulting substance was purified by silica gel column chromatography to obtain the target product.

The yield in grams was 1.06 g, and the percent yield was 88%.

Working Example 4: Synthesis of 2-(4-chlorobenzyl) cyclopentanone N,N-dimethylacetamide (1 mL) and pyridine (0.76 mL, 0.00375 × 2.5 mol) were added to 3-(4-chlorobenzyl)-2-oxocyclopentane carboxylic acid methyl ester (1.0 g, 0.00375 mol), and then acetic acid (0.54 mL, 0.00375 × 2.5 mol) was added. An isobaric dropping funnel was mounted between a cooling condenser and the reaction vessel, and they were stirred at 155°C for 7 hours such that the distillation product did not return. Ethyl acetate and saturated sodium bicarbonate water were added to the reaction solution, and after the mixture was partitioned, the aqueous layer was extracted with ethyl acetate. The organic layer was dried with anhydrous sodium sulfate and concentrated, and then the resulting substance was purified by silica gel column chromatography to obtain the target product. The yield in grams was 0.75 g, and the percent yield was 96%.

### Working Example 5: Synthesis of 1-(4-chlorophenyl)ethanone

N,N-dimethylacetamide (1 mL) and pyridine (1.6 mL, 0.0047 × 2.5 mol) were added to 3-(4-chlorophenyl)-3-oxopropionic acid methyl ester (1.0 g, 0.0047 mol), and then acetic acid (0.67 mL, 0.0047 × 2.5 mol) was added. In an argon atmosphere, an isobaric dropping funnel was mounted between a cooling condenser and the reaction vessel, and they were reacted at 155°C for 9 hours such that the distillation product did not return. Ethyl acetate and saturated sodium bicarbonate water were added to the reaction solution, and after the mixture was partitioned, the aqueous layer was extracted with ethyl acetate. The organic layer was dried with anhydrous sodium sulfate and concentrated, and then the resulting substance was purified by silica gel column chromatography to obtain the target product.

The yield in grams was 0.64 g, and the percent yield was 88%.

### Comparative Example 1: Synthesis of 5-(4-chlorobenzyl)-2,2-bis((methoxymethoxy)methyl) cyclopentanone

In isopropanol (5.5 mL), 1-(4-chlorobenzyl)-3,3-bis((methoxymethoxy)methyl)-2-oxocyclopentane carboxylic acid methyl ester (2.2895 g, 5.52 mmol) was dissolved, and a 2 M sodium hydroxide solution (5.5 mL) was added thereto. The mixture was stirred at 90°C for 2 hours. Following the completion of the reaction, water was added, and the mixture was subjected to extraction using ethyl acetate. The organic layer was washed with saturated brine solution and water, and dried using anhydrous sodium sulfate. The solvent was distilled out, and the residue was purified using silica gel to obtain a target product.

The yield in grams was 1.3029 g, and the percent yield was 66%.

### Reference Example 1: Synthesis 1 of 2-(4-chlorobenzyl)-8,8-dimethyl-7,9-dioxaspiro[4.5]decan-1-one

Toluene (1 mL) was added to 2-(4-chlorobenzyl)-8,8-dimethyl-1-oxo-7,9-dioxaspiro[4.5]decane-2-carboxylic acid methyl ester (10.0 g, 0.0273 mol), and then the resulting mixture was suspended in 0.5 M sodium hydroxide aqueous solution (27.3 mL, 0.0273 mol × 0.5 mol), and reacted at 110°C. The reaction was continued by adding a 0.5 M sodium hydroxide solution (27.3 mL, 0.0273 mol × 0.5 mol) every 2 hours (for three times total), and heating and stirring were performed for total of 9 hours. Following the completion of the reaction, the mixture was allowed to cool to room temperature and then subjected to extraction using toluene. The organic layer was washed with saturated brine solution, and then dried using anhydrous sodium sulfate. Thereafter, the dried and concentrated product was purified using a silica gel column, and a target product was obtained.

The yield in grams was 6.75 g, and the percent yield was 80%.

### Industrial Applicability

The present invention can be used in the production of a 2-benzyl-5,5-di(protected hydroxymethyl)-cyclopentanone derivative serving as a raw material for an agricultural chemical or the like.

## Claims

1. A method of producing a carbonyl compound represented by general formula (I) below from a compound represented by general formula (II) below;
the method comprising a step of subjecting the compound represented by general formula (II) below to dealkoxycarbonylation in the presence of an organic carboxylic acid salt of a tertiary amine: 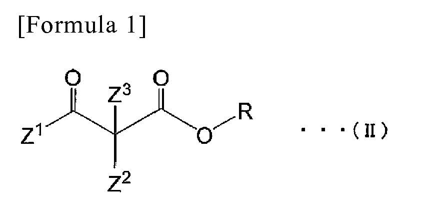 in general formula (II), Z¹ represents a substituted or unsubstituted alkyl group, cycloalkyl group, aryl group, or heterocyclic group; Z³ and Z² each independently represent a hydrogen atom, a substituted or unsubstituted alkyl group, cycloalkyl group, aryl group, or heterocyclic group; and R represents an alkyl group having from 1 to 4 carbons; Z¹ and Z² may be bonded to each other; 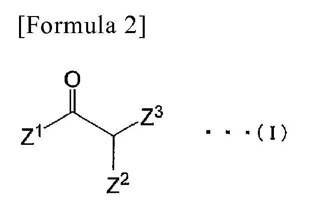 in general formula (I), Z¹, Z², and Z³ are the same as Z¹, Z², and Z³ in general formula (II) above, respectively.

2. The method of producing a carbonyl compound according to claim 1, wherein a tertiary amine constituting the organic carboxylic acid salt of a tertiary amine is triethylamine, trimethylamine, pyridine, or picoline.

3. The method of producing a carbonyl compound according to claim 1 or 2, wherein an organic carboxylic acid constituting the organic carboxylic acid salt of a tertiary amine is an aliphatic monocarboxylic acid.

4. The method of producing a carbonyl compound according to any one of claims 1 to 3, wherein the organic carboxylic acid salt of a tertiary amine is acetate of triethylamine or pyridine.

5. The method of producing a carbonyl compound according to any one of claims 1 to 4, wherein an alkali metal salt of an organic carboxylic acid is further added in the reaction system.

6. The method of producing a carbonyl compound according to any one of claims 1 to 5, wherein the compound represented by general formula (II) above is a compound represented by general formula (IIa) below; and
the carbonyl compound represented by general formula (I) above is a carbonyl compound represented by general formula (Ia) below: 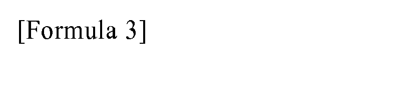 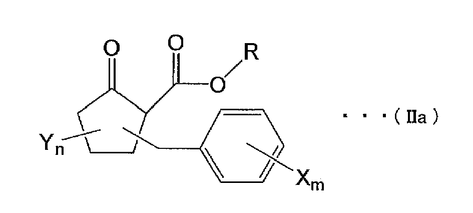 in general formula (IIa), R represents an alkyl group having from 1 to 4 carbons, Y represents an alkyl group or haloalkyl group having from 1 to 6 carbons, an alkenyl group or haloalkenyl group having from 2 to 6 carbons, an alkynyl group or haloalkynyl group having from 2 to 6 carbons, or a group in which a part of hydrogen atoms of the alkyl group, haloalkyl group, alkenyl group, haloalkenyl group, alkynyl group, or haloalkynyl group is substituted with -OG (G represents a protecting group of a hydroxy group); and n is an integer from 0 to 6; when n is 2 or greater, a plurality of Y may be the same or different; when n is 2 or greater, a plurality of Y may be bonded to each other and, together with carbon atom(s) to which the plurality of Y are bonded, form a ring; X represents a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; and m is an integer from 0 to 5, when m is 2 or greater, a plurality of X may be the same or different; 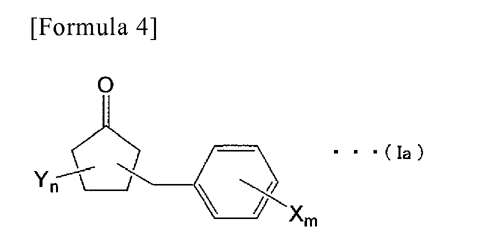 in general formula (Ia), X, Y, m, and n are the same as X, Y, m, and n in general formula (IIa) above, respectively.

7. The method of producing a carbonyl compound according to claim 6, wherein the compound represented by general formula (IIa) above is a compound represented by general formula (IIb) below; and
the carbonyl compound represented by general formula (Ia) above is a carbonyl compound represented by general formula (Ib) below: 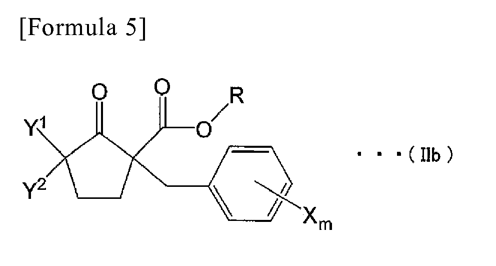 in general formula (IIb), Y¹ and Y² each independently represent an alkyl group or haloalkyl group having from 1 to 6 carbons, or a group in which a part of hydrogen atoms of the alkyl group or haloalkyl group is substituted with -OG¹ (G¹ represents a protecting group of a hydroxy group), and Y¹ and Y² may be bonded to each other and, together with a carbon atom to which Y¹ and Y² are bonded, form a ring; R, X, and m are the same as R, X, and m in general formula (IIa) above, respectively; 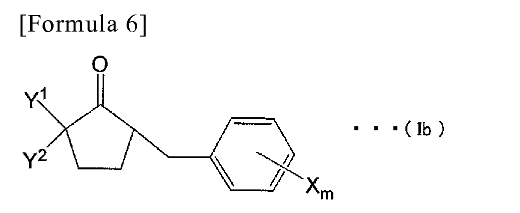 in general formula (Ib), X, Y¹, Y², and m are the same as X, Y¹, Y², and m in general formula (IIb) above, respectively.

8. The method of producing a carbonyl compound according to claim 7, wherein the compound represented by general formula (IIb) above is a compound represented by general formula (IIc) below; and
the carbonyl compound represented by general formula (Ib) above is a carbonyl compound represented by general formula (Ic) below: 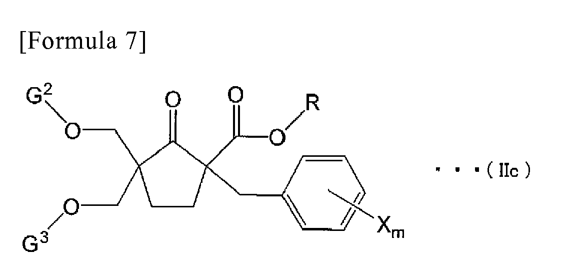 in general formula (IIc), G² and G³ each independently represent a protecting group that dissociates under acidic conditions, and G² and G³ may be bonded to each other; R, X, and m are the same as R, X, and m in general formula (IIa) above, respectively; 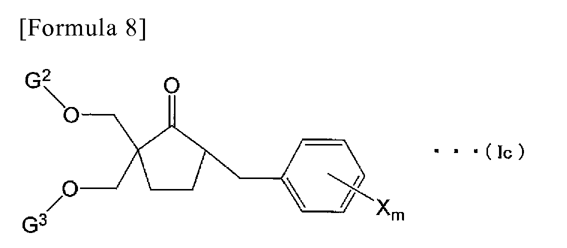 in general formula (Ic), X, G², G³, and m are the same as X, G², G³, and m in general formula (IIc) above, respectively.

9. The method of producing a carbonyl compound according to claim 8, wherein the organic carboxylic acid salt of a tertiary amine is produced in a reaction system by adding a tertiary amine to a compound represented by general formula (IIc) above and then adding an organic carboxylic acid in an amount smaller than the added amount of the tertiary amine, and dealkoxycarbonylation is performed.

10. The method of producing a carbonyl compound according to claim 8 or 9, wherein the compound represented by general formula (IIc) above is a compound represented by general formula (IId) below; and
the carbonyl compound represented by general formula (Ic) above is a carbonyl compound represented by general formula (Id) below: 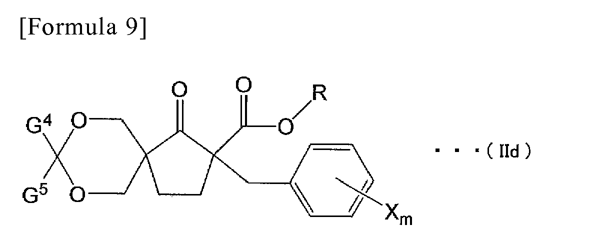 in general formula (IId), G³ and G⁵ each independently represent a hydrogen atom, an alkyl group having from 1 to 4 carbons, an alkenyl group having from 1 to 4 carbons, a substituted or unsubstituted phenyl group, naphthyl group, or benzyl group, and G⁴ and G⁵ may be bonded to each other and, together with a carbon atom to which G⁴ and G⁵ are bonded, form a ring;R, X, and m are the same as R, X, and m in general formula (IIa) above, respectively; 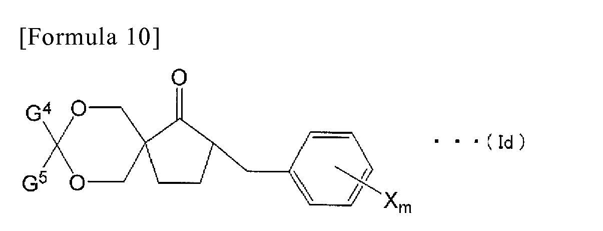 in general formula (Id), X, G⁴, G⁵, and m are the same as X, G⁴, G⁵, and m in general formula (IId) above, respectively.

11. The method of producing a carbonyl compound according to claim 10, wherein G⁴ and G⁵ are each independently a hydrogen atom or an alkyl group having from 1 to 4 carbons.

12. The method of producing a carbonyl compound according to any one of claims 6 to 11, wherein m is an integer from 0 to 2; and when m is 1 or 2, X is a halogen atom.
